(19) Europäisches Patentamt
European Patent Office
Office européen des brevets

(11) Publication number: **0 526 541 B1**

(12) **EUROPEAN PATENT SPECIFICATION**

(45) Date of publication of patent specification: **25.01.95**

(51) Int. Cl.⁶: **A61K 33/30**, A61K 31/19, A61L 15/18, A61L 15/20

(21) Application number: **91908641.3**

(22) Date of filing: **23.04.91**

(86) International application number: **PCT/SE91/00288**

(87) International publication number: **WO 91/16059 (31.10.91 91/25)**

(54) **MEANS HAVING MICROBIAL EFFECT AND LITTLE OR COMPLETELY ABOLISHED TENDENCY TO DEVELOP CONTACT ALLERGIC REACTIONS OR TOXIC EFFECTS AND USE THEREOF IN E.G. SKIN AND WOUND TREATMENT PRODUCTS.**

(30) Priority: **24.04.90 SE 9001475**

(43) Date of publication of application:
**10.02.93 Bulletin 93/06**

(45) Publication of the grant of the patent:
**25.01.95 Bulletin 95/04**

(84) Designated Contracting States:
**BE DE DK ES FR GB IT NL SE**

(56) References cited:
**EP-A- 0 153 915**
**GB-A- 1 395 815**

**Patent Abstracts of Japan, Vol.11, No 262, C442,abstract of JP 62-63518, publ 1987-03-20ARAKAWA CHEM IND CO LTD**

(73) Proprietor: **MÖLNLYCKE AKTIEBOLAG**

**S-405 03 Göteborg (SE)**

(72) Inventor: **SÖDERBERG, Thor**
**Bölevägen 38**
**S-902 52 Umeaa (SE)**
Inventor: **HOLM, Stig**
**Norra Gimonäsvägen 25**
**S-902 40 Umeaa (SE)**
Inventor: **HALLMANS, Göran**
**Granvägen 8 B**
**S-902 40 Umeaa (SE)**

(74) Representative: **Hyltner, Jan-Olof et al**
**Noréns Patentbyra AB**
**P.O. Box 27034**
**S-102 51 Stockholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Patent Abstracts of Japan, Vol 8, No 168, C236,abstract of JP 59-67219, publ 1984-04-16SEISAN KAIHATSU KAGAKU KEN-KYUSHO

Pastent Abstract of Japan, Vol.6, No.266, C142, abstract of JP 57-158724, publ 1982-09-30 SHISEIDO K.K.

## Description

The invention relates to an agent that possesses antimicrobial properties and exhibits a minimum tendency, or a completely discontinued tendency, to generate contact allergic reactions or toxic effects. The invention relates in particular to an agent which because of its antimicrobial properties is suitable for use as a curative constituent of products intended for the treatment of the skin, wounds and mucus-membrane, for example wound dressings and the like, and also for use in, for instance, rinsing solutions for medical use. The invention also relates to the use of said agent in the manufacture of such products, such as the manufacture of wound dressings or the like, or in the manufacture of a rinsing solution for medicinal use.

Background Art

Zinc compounds have long been used in the treatment of wounds. The bacteria-inhibiting and local-drying properties of zinc are well known. Colophony has also been earlier used for its bacteria-inhibiting properties.

Colophony is obtained from certain Pinus species and consists of about 90% free resin acids, with the remainder consisting of neutral substances, oxidized terpentines and minor quantities of esters and anhydrides, depending upon the origin of the colophony. The resin acids are a group of mono-basic carboxylic acids, all with a phenanthrene skeleton and all having 20 carbon atoms in the molecule. With two exceptions, dextro-pimaric acids, the resin acids exhibit the same substituents in the same positions and differ only with respect to the number and the positions of the double bonds.

It is also known to use zinc compounds in combination with colophony in, for instance, zinc tape intended for wound care. MEZINC[R] (from Mölnlycke AB), which is an occlusive dressing consisting of cotton fabric coated with PVC and an adhesive substance consisting of rubber, mineral oils, colophony and Zn O possesses good bacteria inhibiting properties against various bacterial strains. In this case, zinc compounds and colophony are used for their respective bacteria inhibiting effect and colophony is also used as a mediator for the transportation of zinc ions to tissue and, in the adhesive substance, also for its adhesive properties.

One disadvantage with the use of adhesive products which contain zinc compounds in combination with colophony is that these products engender contact-allergic reactions in many people, which has reduced the use of these adhesive products. It is true that when used, the zinc present in the adhesive product will neutralize or inactivate the major part of the colophony present. The contact allergic reactions remain, however, to a large extent.

It is known from Patent Abstract of Japan, Vol. II, No. 262, C-442, abstract of JP62-63518, publ. 20th March, 1987 and Patent Abstract of Japan, Vol. 8, No. 8, C-236, abstract of JP59-67219, publ. 16th April, 1984, that substances or products which contain abietic acid or dehydroabietic acid and/or dihydroabietic acid are effective against passive cutaneous anaphylaxis (PCA), which is a rapid hypersensitive reaction (anaphylactic) which manifests itself in hayfever, urticaria, argio neurotic oedema, atopic eczema, anaphylactic shock or skin rashes within a period of about 10 to 20 minutes. This anaphylactic effect shall be held separate from another type of allergic condition with which the present invention is concerned, namely the classic contact eczema which is effectuated through direct contact between immune cells and cells which have the allergen bound to their surfaces and not by humoral antibodies, this reaction developing within about 12 to 48 hours. The two publications cited above are not concerned with contact allergies but allege an inhibiting effect of the acids against PCA.

GB-A-1,395,815 describes an adsorbent wound dressing material coated with a non-adhesive acrylic resin layer, which comprises a pigment such as zinc oxide.

EP-A2-0,153,915 describes a dressing, which incorporates zinc oxide bound with the aid of a tissue-compatible polymer, for cleaning sores and stimulating the healing thereof.

Patent Abstract of Japan, vol 6, No 266, C142, abstract of JP 57-158724, describes a composition comprising an aqueous solution of zinc oxide solubilized with an amino acid.

Object of the Invention

The object of the invention is to attempt to eliminate the drawbacks associated with known zinc-colophony-based adhesive products and the like and to provide a product which while affording a pronounced bacteria-inhibiting effect will not induce pronounced contact-allergic reactions.

Disclosure of the Invention

This object is surprisingly achieved in accordance with the invention by substituting colophony of conventional zinc-colophony products with one or more substantially pure resin acids, selected from dehydroabietic acid, neoabietic acid, pimaric acid, levopimaric acid, isopimaric acid, palustrinic acid and dehydroabietylamine, in a weight ratio of the components a) and b) of between 1:2 and 20:1, calculated on $Zn^{2+}$ as ZnO and on pure component b). As will be evident from the following, it has been found that such a combination exhibits splendid antimicrobial properties and that both the contact-allergic and the toxic effect have been suppressed to a large extent. The exudation or release of zinc from, for instance, adhesive product to serum through the agency of the acid is improved furthermore.

This was surprising, since the same acids, although in an impure form and together with other substances, are present in colophony, which exhibits pronounced contact-allergic and toxic effects. Although no simple explanation can be given as to why this is so, it is obvious that the purity of the acids is significant to the good effect achieved. The acid or acids present in the agent shall have the purest possible form and preferably be at least 98% pure, and even more preferably at least 99% pure. However, the aforesaid effects have also been obtained with acids having a purity of about 40-60%. As will be seen from the following test reports on the resin acids used, dehydroabietic acid has been found to be the best of these acids because it provides the best bacteria inhibiting and toxicity lowering low-allergenic effect when used together with zinc. The zinc component may be present in the form of a compound which produces $Zn^{2+}$-ions when the agent is used to treat wounds. Zn O is the most usual compound, although salts with inorganic acids, such as Zn $SO_4$ and Zn $Cl_2$ can also be used to the same effect.

The zinc component and the acid component are present in the inventive agent in proportions of at least 1:2 and at most of 20:1, based on the weight of zinc components as Zn O and on one pure acid component. When proportions greater than 20:1 are used, the effect of the acid component on the agent when treating wounds is excessively low, whereas proportions lower than 1:2 do not improve the effect of the agent and the agent becomes unnecessarily expensive, because the acid is much more expensive than the zinc component and the release of zinc through the intermediary of the acid is affected negatively.

The aforesaid properties of the inventive agent render the agent particularly suitable for use in different skin and wound treatment products, and also in products intended for the treatment of mucus-membrane, such as mouth mucus-membrane, stomach mucus-membrane and intestinal mucus-membrane. Accordingly, the invention also relates to the use of the inventive agent in the preparation of such a product, for example a wound care product. An example of one such product is an adhesive wound dressing whose manufacture includes the use of conventionally used components, such as solvent, mineral oil, rubber solution, etc., for instance. In addition, the agent can also be used in building materials (fibreboard), insulating materials, paper, and surface treating materials (such as paints), thereby greatly improving such products with respect to their antimicrobial, contact-allergic and toxic effects. In this regard, calcium and magnesium ions may also be included, these ions playing the same part as the zinc ions.

The invention will now be described in more detail with reference to the accompanying drawings, in which

Figure 1A shows the antimicrobial effect in the form of inhibiting zones in mm as a function of time in the case of the known zinc and colophony combination;

Figure 1B illustrates the effect achieved when solely zinc is present in various concentrations, in the absence of an acid component;

Figure 1C illustrates the effect obtained when using seven different resin acids in accordance with the invention;

Figure 1D illustrates the effect obtained when using a combination of zinc oxide and abietic acid in accordance with the invention, in different weight ratios;

Figure 1E illustrates the effect obtained when using a combination of zinc oxide and dehydroabietic acid in accordance with the invention in a mixing ratio of 16:1; and

Figure 2 illustrates cytotoxicity expressed as $^{51}$Cr-leakage as a function of the amount of dehydroabietic acid (DHAA) present with and without the presence of a zinc component.

Antimicrobial (Antibacterial) Effect:

The nature of the experiment:
0.1 ml of a suspension ($1.10^5$) bacteria/ml) of bacteria (Staphylococcus Aureus S-209) in log-phase-growth were spread with the aid of glass beads over an agar plate in a plastic tray, such as to obtain uniform growth. An adhesive product in the form of pieces of adhesive tape were applied in a circular pattern to the agar gel, with the aid of sterilized pincettes. Incubation was effected in a heated cabinet at 37°C. The inhibition zones were recorded for a full test series after 12, 24, 48 and 72 hours, with the aid of an mm-graduated rule placed against the edges of the plastic tray, with an eye-distance to the tray of about 40 cm and at an accuracy of 0.5 mm.

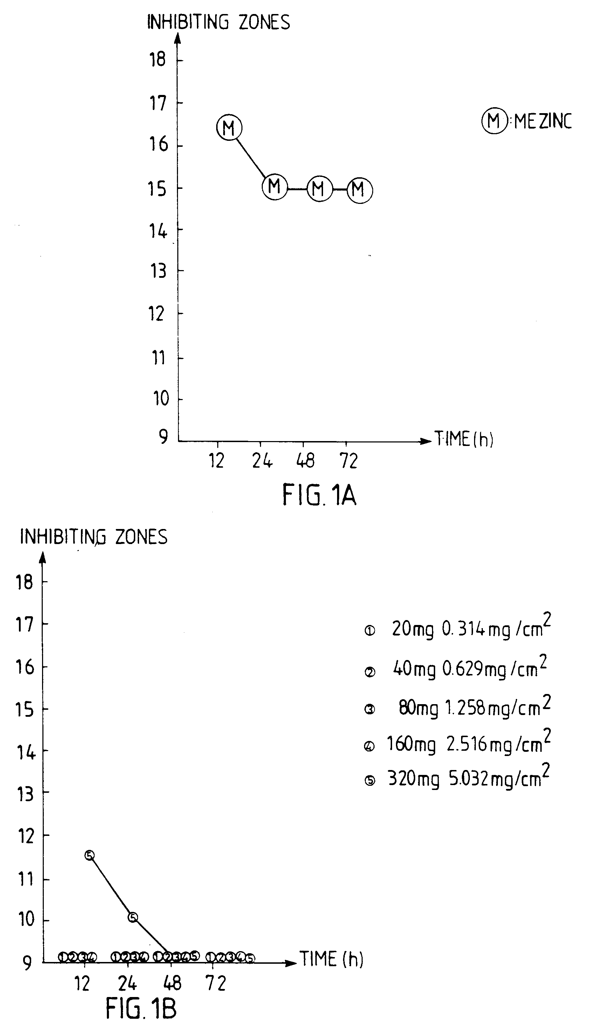

a) There was first tested an adhesive product comprising the known product MEZINC[R] (Mölnlycke AB) containing zinc oxide (2.4 mg/cm$^2$), colophony (3.04 mg/cm$^2$) and natural rubber (2.28 mg/cm$^2$). The result recorded is shown in Figure 1A.

b) Also tested was an adhesive product which contained Zn O in various concentrations, but no resin acid component at all. The result is illustrated in Figure 1B.

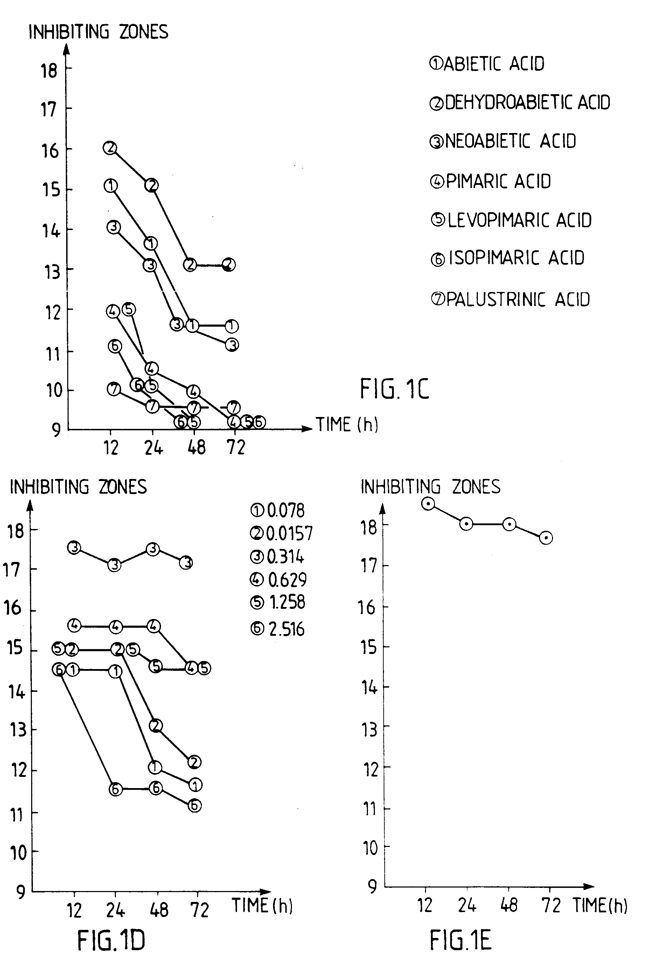

c) Seven mutually different adhesive products were tested, each containing 1.258 mg/cm$^2$ of abietic acid, dehydroabietic acid, neoabietic acid, pimaric acid, levopimaric acid, isopimaric acid and palustrinic acid respectively. The results are given in Figure 1C.

d) Also tested was an adhesive product containing zinc oxide and abietic acid in the weight ratios of 2:1, 4:1, 8:1, 16:1, 32:1 and 64:1, Nos. 6, 5, 4, 3, 2 and 1, respectively. The results are set forth in Figure 1D.

e) Also tested was an adhesive product containing Zn O and dehydroabietic acid in the weight ratio of 16:1. The results are seen in Figure 1E.

Conclusion:

The results of these experiments or tests show that a) solely Zn O fails to provide a particularly high anti-bacterial effect, b) that the individual acids provide a much higher antibacterial effect, and c) that the combination of highly pure acid and zinc oxide provides an antibacterial effect which is at least equal to the effects obtained with an adhesive product that contains colophony and zinc oxide.

Allergic Effect:

Test method: A so-called patch test was carried out while using Finn Chambers[R] (Fregert S. Manual of Contact Dermatitis, 2nd ed., Copenhagen: Munksgaard 1981; 71-81) on a total of 14 patients suffering from known contact allergy against colophony. Highly pure resin acids, namely abietic acid (90-95% pure), dehydroabietic acid (> 99%) and isopimaric acid (> 99%) (from Helix Biotech Ltd., Richmond, B.C., Canada) were tested. The reactions against the actual adhesive product itself (the same as MEZINC[R]), against the zinc oxide and against the pure resin acids were established and are set forth in the following Table 1.

Conclusion: The allergy tests showed that out of 14 patients with known contact allergy against colophony, very few showed any of the same allergic reaction against highly pure individual acids and zinc oxide. This shows the superior effect of an agent produced in accordance with the invention.

Toxic Effect:

The cytotoxic effect in vitro was studied for dehydroabietic acid, a representative compound used in accordance with the invention, on human polymorphoneuclear leucocytes (PMN-cells) while measuring the leakage of $^{51}$Cr from labelled cells. In these tests, cell membrane damage was manifested in an increase in plasma membrane permeability, resulting in an increased leakage of cytoplasmic localized $^{51}$Cr.

Preparation of PMN cells for the purpose of assessing cytotoxicity: Venous blood was taken from adult volunteers in acid-citrate-dextrose-tubes. The granulocytes were isolated from the erythrocytes by dextran sedimention, essentially in accordance with Babior and Cohen (Cline M.J. (ed): Leucocyte function: New York: Churchill-Livingstone, 1981:1-38). The leucocytic cell mass was centrifuged at 160xg for 10 minutes at 4°C. The supernatant liquid was thrown away and remaining erythrocytes were eliminated by adding ice cold distilled water. After 30 seconds the toxicity was re-established with ice cold 0.6 M Na Cl. The cells were washed three times (160xg, for 5 minutes at 4°C) and finally dispersed in ice cold RPMI, $10^7$ cells/ml. 90% of the cell suspension consisted of PMN-cells.

The cell suspension was incubated with Na $^{51}$Cr RO$_4$ (90 $\mu$ Ci/ml, de Pont de Nemours, Dreieich, Federal Republic of Germany) for 60 minutes at 37°C, washed four times (160xg, 5 minutes at 4°C) and dispersed in modified GBSS (see below) to $10^7$ cells/ml.

The zinc source used was a filtrate of Zn O produced by preparing a zinc oxide suspension of 179 mg Zn O (Merck, Darmstadt, Federal Republic of Germany) and 100 ml of modified Gey's solution (modified GBSS consisting of 8 g Na Cl, 0.37 g KCl, 0.07 g MgSO$_4$ • 7H$_2$O, 0.21 g MgCl$_2$ • 6H$_2$O, 0.22 g MgCl$_2$ • 2H$_2$O, 1.0 g glucose and 2.38 g HEPES added with 1000 ml water, pH 7.3) and stirred for 20 hours at 37°C. The suspension was passed through a 0.2 $\mu$m membrane filter and the filtrate was used in the experiment. Dehydroabietic acid (DHAA) was used in the form of a storage solution of DHAA (purity > 99% from Helix Biotech Ltd., Vancouver, B.C., Canada) prepared by dissolving 100 mg DHAA in 1 ml of 99% ethanol.

The test solutions were prepared 30 minutes prior to adding the PMN-cells and consisted of DHAA: 0-500 $\mu$g/ml, with and without zinc oxide as the aforesaid filtrate, 300 $\mu$mol/l.

50 $\mu$l of $^{51}$Cr labelled PMN-cells were mixed with 100 $\mu$l of the test solutions on a fabric cultivating plate having 96 wells (Linbro Flow Lab) in triple arrays. Six wells on each plate were used without a DHAA addition, with or without zinc oxide filtrate, as a comparison test. Subsequent to filling the wells with the test solutions, the plates were incubated for 60 minutes at 37°C, and were then centrifuged at 500xg for 5 minutes and the radio activity (counts per 120 s) was measured in 75 $\mu$l supernatant liquid in a gamma-scintillation spectrometer (Auto-gamma Scintillation Spectrometer Packard). The percentual release of $^{51}$Cr, indicating cell membrane damage, was calculated with the aid of the following equation:

$$\text{\% cytotoxicity} = \frac{\text{leakage} - \text{spontaneous leakage}}{\text{maximum leakage} - \text{spontaneous leakage}} \cdot 100$$

Spontaneous leakage never exceeded 10% of maximum leakage.

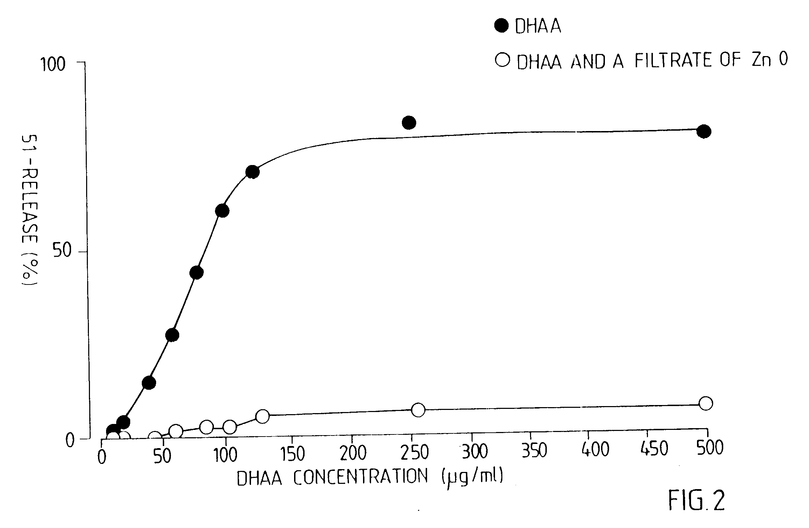

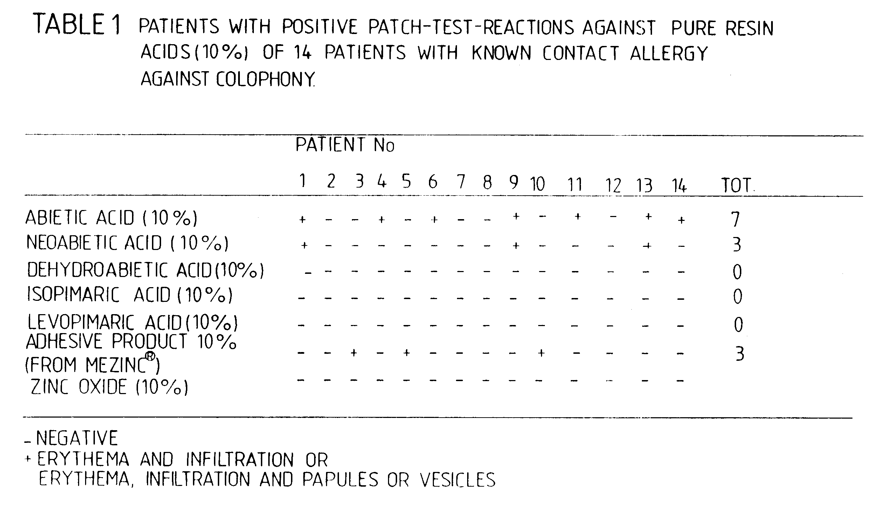

The result: The result is set forth in Figure 2. Toxic effects on the PMN-cells were observed at low concentrations of DHAA (20 $\mu$g/ml). A marked increase in toxicity was then observed up to 250 $\mu$g/ml DHAA. When the zinc oxide filtrate was used together with DHAA, no toxic effect of DHAA was observed up to 100 $\mu$g/ml. Only slight toxicity was registered from about 125 $\mu$g/ml.

Thus, in summary, the inventive agent exhibits splendid, unexpected properties which render the agent well suited for use in different skin, wound and mucus-membrane treatment products and also in such products as building material (fibreboard and insulating material), paper and surface treatment materials (paints and the like).

## Claims

1. An agent having an antimicrobial effect and no or a minimum tendency of producing contact allergic reactions or toxic effects, **characterized** in that the agent consists of a mixture of a) a compound which produces Zn$^{2+}$ -ions and b) one or more substantially pure resin acids selected from dehydroabietic acid, neoabietic acid, pimaric acid, levopimaric acid, isopimaric acid, palustrinic acid and dehydroabietylamine, in a weight ratio of the components a) and b) of between 1:2 and 20:1, calculated on Zn$^{2+}$ as ZnO and on pure component b).

2. An agent according to Claim 1 for use in a skin or wound treatment product, such as a wound dressing, a rinsing solution for medicinal use or for treating the oral cavity/teeth.

3. An agent according to Claims 1 and 2, **characterized** in that the Zn$^{2+}$-ions producing compound is ZnO.

4. An agent according to Claims 1-3, **characterized** in that the agent is a mixture of a) a compound which produces Zn$^{2+}$ ions and b) dehydroabietic acid.

5. Wound dressing, **characterized** by having a layer comprising an agent consisting of a mixture of a) a compound which produces Zn$^{2+}$-ions and b) one or more substantially pure resin acids selected from dehydroabietic acid, neoabietic acid, pimaric acid, levopimaric acid, isopimaric acid, palustrinic acid, and dehydroabietylamine, in a weight ratio of the components a) and b) of between 1:2 and 20:1, calculated on Zn$^{2+}$ as ZnO and on pure component b).

6. The use of the agent according to Claim 1 consisting of a mixture of a) a compound which produces Zn$^{2+}$-ions and b) one or more substantially pure resin acids selected from dehydroabietic acid, neoabietic acid, pimaric acid, levopimaric acid, iso-pimaric acid, palustrinic acid, and dehydroabietylamine, in a weight ratio of the components a) and b) of between 1:2 and 20:1, calculated on Zn$^{2+}$ as ZnO and on pure component b), in such products as insulating material, building material, paper, surface treatment material, paints, glues, cleaning agents, disinfectants, cosmetics which contain conventionally different types of resins.

**7.** The use according to Claim 6, wherein calcium ions and/or magnesium ions are also present.

## Patentansprüche

**1.** Mittel mit antimikrobieller Wirkung und keiner oder einer geringen Tendenz zur Herbeiführung von kontaktallergischen Reaktionen oder toxischen Wirkungen, dadurch gekennzeichnet, daß das Mittel auf einer Mischung aus a) einer Verbindung, die $Zn^{2+}$-Ionen bildet, und b) einer oder mehreren, im wesentlichen reinen Harzsäuren, die unter Dehydroabietinsäure, Neoabietinsäure, Pimarsäure, Levopimarsäure, Isopimarsäure, Palustrinsäure und Dehydroabietylamin ausgewählt sind, besteht, wobei das Gewichtsverhältnis der Komponenten a) und b) 1:2 bis 20:1 beträgt, berechnet auf der Basis von $Zn^{2+}$ in Form Von ZnO und auf der Basis der reinen Komponente b).

**2.** Mittel nach Anspruch 1 zur Verwendung in einem Haut- oder Wundbehandlungsprodukt, z.B. als Wundverbandstoff, als Spüllösung für medizinische Zwecke oder zur Behandlung der Mundhöhle/Zähne.

**3.** Mittel nach Anspruch 1 und 2, dadurch gekennzeichnet, daß es sich bei der $Zn^{2+}$-Ionen bildenden Verbindung um ZnO handelt.

**4.** Mittel nach Anspruch 1 bis 3, dadurch gekennzeichnet, daß es sich bei dem Mittel um eine Mischung von a) einer Verbindung, die $Zn^{2+}$-Ionen bildet, und b) Dehydroabietinsäure handelt.

**5.** Wundverbandstoff, gekennzeichnet durch eine Schicht, die ein Mittel enthält, das aus einer Mischung von a) einer Verbindung, die $Zn^{2+}$-Ionen bildet, und b) einer oder mehreren, im wesentlichen reinen Harzsäuren, die unter Dehydroabietinsäure, Neoabietinsäure, Pimarsäure, Levopimarsäure, Isopimarsäure, Palustrinsäure und Dehydroabietylamin ausgewählt sind, besteht, wobei das Gewichtsverhältnis der Komponenten a) und b) 1:2 bis 20:1 beträgt, berechnet auf der Basis von $Zn^{2+}$ in Form Von ZnO und auf der Basis der reinen Komponente b).

**6.** Verwendung des Mittels nach Anspruch 1, das aus einer Mischung von a) einer Verbindung, die $Zn^{2+}$-Ionen bildet, und b) einer oder mehreren, im wesentlichen reinen Harzsäuren, die unter Dehydroabietinsäure, Neoabietinsäure, Pimarsäure, Levopimarsäure, Isopimarsäure, Palustrinsäure und Dehydroabietylamin ausgewählt sind, besteht, wobei das Gewichtsverhältnis der Komponenten a) und b) 1:2 bis 20:1 beträgt, berechnet auf der Basis von $Zn^{2+}$ in Form von ZnO und auf der Basis der reinen Komponente b), in Produkten, wie Isoliermaterialien, Baustoffen, Papier, Oberflächenbehandlungsmaterialien, Anstrichmitteln, Klebstoffen, Reinigungsmitteln, Desinfektionsmitteln und Kosmetika, die herkömmlicherweise unterschiedliche Typen von Harzen enthalten.

**7.** Verwendung nach Anspruch 6, wobei ferner Calciumionen und/oder Magnesiumionen vorhanden sind.

## Revendications

**1.** Agent ayant un effet antimicrobien et aucune tendance, ou alors une tendance minimale, à engendrer des effets toxiques ou des réactions allergiques par contact, caractérisé en ce qu'il est constitué d'un mélange de a) un composé qui produit des ions $Zn^{2+}$ et b) un ou plusieurs acides résiniques sensiblement purs, choisis parmi l'acide déshydroabiétique, l'acide néoabiétique, l'acide pimarique, l'acide lévopimarique, l'acide isopimarique, l'acide palustrinique et la déshydroabiétylamine, en un rapport pondéral des composants a) et b) compris entre 1:2 et 20:1, calculé sur $Zn^{2+}$ sous forme de ZnO et sur le composant b) pur.

**2.** Agent suivant la revendication 1 pour l'utilisation dans un produit de traitement de la peau ou d'une blessure, comme un pansement de blessure, une solution de rinçage à l'usage médical, ou pour le traitement des dents/cavité buccale.

EP 0 526 541 B1

3. Agent suivant l'une quelconque des revendications 1 et 2, caractérisé en ce que le composé qui produit des ions $Zn^{2+}$ est le ZnO.

4. Agent suivant l'une quelconque des revendications 1 à 3, caractérisé en ce qu'il est constitué d'un mélange de a) un composé qui produit des ions $Zn^{2+}$ et b) l'acide déshydroabiétique.

5. Pansement pour blessure, caractérisé en ce qu'il comporte une couche comprenant un agent constitué d'un mélange de a) un composé qui produit des ions $Zn^{2+}$ et b) un ou plusieurs acides résiniques sensiblement purs, choisis parmi l'acide déshydroabiétique, l'acide néoabiétique, l'acide pimarique, l'acide lévopimarique, l'acide isopimarique, l'acide palustrinique et la déshydroabiétylamine, en un rapport pondéral des composants a) et b) compris entre 1:2 et 20:1, calculé sur $Zn^{2+}$ sous forme de ZnO et sur le composant b) pur.

6. Utilisation de l'agent suivant la revendication 1, constitué d'un mélange de a) un composé qui produit des ions $Zn^{2+}$ et b) un ou plusieurs acides résiniques sensiblement purs, choisis parmi l'acide déshydroabiétique, l'acide néoabiétique, l'acide pimarique, l'acide lévopimarique, l'acide isopimarique, l'acide palustrinique et la déshydroabiétylamine, en un rapport pondéral des composants a) et b) compris entre 1:2 et 20:1, calculé sur $Zn^{2+}$ sous forme de ZnO et sur le composant b) pur dans des produits comme des matériaux isolants, des matériaux de construction, le papier, un matériau de traitement de surfaces, des peintures, des colles, des agents de nettoyage, des désinfectants, des produits cosmétiques, qui contiennent classiquement différents types de résines.

7. Utilisation suivant la revendication 6, caractérisée en ce que des ions calcium et/ou des ions magnésium sont également présents.

8

FIG. 1A

FIG. 1B

9

INHIBITING ZONES

① ABIETIC ACID

② DEHYDROABIETIC ACID

③ NEOABIETIC ACID

④ PIMARIC ACID

⑤ LEVOPIMARIC ACID

⑥ ISOPIMARIC ACID

⑦ PALUSTRINIC ACID

TIME (h)

FIG. 1C

INHIBITING ZONES

① 0.078
② 0.0157
③ 0.314
④ 0.629
⑤ 1.258
⑥ 2.516

TIME (h)

FIG.1D

INHIBITING ZONES

TIME(h)

FIG.1E

10

FIG.2

EP 0 526 541 B1

# TABLE 1 PATIENTS WITH POSITIVE PATCH-TEST-REACTIONS AGAINST PURE RESIN ACIDS (10%) OF 14 PATIENTS WITH KNOWN CONTACT ALLERGY AGAINST COLOPHONY.

| | PATIENT No | | | | | | | | | | | | | | TOT. |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | |
| ABIETIC ACID (10%) | + | − | − | + | − | + | − | − | + | − | + | − | + | + | 7 |
| NEOABIETIC ACID (10%) | + | − | − | − | − | − | − | − | + | − | − | − | + | − | 3 |
| DEHYDROABIETIC ACID (10%) | − | − | − | − | − | − | − | − | − | − | − | − | − | − | 0 |
| ISOPIMARIC ACID (10%) | − | − | − | − | − | − | − | − | − | − | − | − | − | − | 0 |
| LEVOPIMARIC ACID (10%) | − | − | − | − | − | − | − | − | − | − | − | − | − | − | 0 |
| ADHESIVE PRODUCT 10% (FROM MEZINC®) | − | − | + | − | + | − | − | − | − | + | − | − | − | − | 3 |
| ZINC OXIDE (10%) | − | − | − | − | − | − | − | − | − | − | − | − | − | − | |

− NEGATIVE
+ ERYTHEMA AND INFILTRATION OR
  ERYTHEMA, INFILTRATION AND PAPULES OR VESICLES

EP 0 526 541 B1